# EUROPEAN PATENT APPLICATION

(11) **EP 4 837 500 A1**
(43) Date of publication of application: **11.11.2026**
(21) Application number: 23961301.1
(22) Date of filing: 12.12.2023
(51) Int. Cl.: A61B 5/00, A01K 11/00, A01K 29/00

(54) **DEVICE FOR REMOTELY MONITORING BODY TEMPERATURE IN ANIMALS**

(71) Applicant: Veterinaria de Precisión Diagnóstica S.L., 16413 Torrubia Del Campo (Cuenca) (ES)
(72) Inventor: ESPINOSA CRESPO, Ignacio Antonio, 16413 TORRUBIA DEL CAMPO (CUENCA) (ES)
(74) Representative: Falcon Morales, Alejandro
(86) International application number: PCT/ES2023/070738
(87) International publication number: WO 2025/125696

(57) **Abstract**

A remote animal-temperature monitoring device, comprising a housing (1) which in turn comprises a hollow interior (2) and a cover (3), the cover (3) comprising a first region (7) configured to be inserted into the housing (1) and comprising grooves wherein seals (10) are located, the seals (10) being configured to provide fluid-tightness, and a second region (8) opposite the first region (7); and a core (4) housed in the hollow interior (2) of the housing (1), the core (4) comprising a self-programmable circuit (12), a memory, a temperature probe (13) responsible for measuring temperature and for sending this information to the self-programmable circuit (12), and a battery (16).

## Description

### TECHNICAL FIELD

The present invention relates to a remote animal-temperature monitoring device, the device being configured to measure the animal's body temperature and heart rate in real time, to measure the pH of various regions of the animal in real time, and to transmit those data remotely. The device forming the subject matter of the invention is applicable in the field of veterinary medicine and livestock health and, more particularly, in the field of diagnostic instruments based on body temperature under intensive farming conditions.

### BACKGROUND OF THE INVENTION

An increase in an animal's body temperature is a common sign of various diseases, many of which are infectious in origin and may have a significant impact on the animal's health and performance. Early identification of such an increase in temperature is essential for improving the diagnosis, treatment and prognosis of the disease in question.

Until now, temperature measurement has been performed at specific points in time using clinical thermometers inserted rectally, without the possibility of continuously monitoring the animal and requiring repeated handling of the animal.

Heart rate is also an important indicator of an animal's health. An abnormal increase or decrease in heart rate may be a sign of disease, stress, pain or dehydration. Monitoring heart rate allows veterinarians and livestock farmers to detect health problems at an early stage and to take measures to treat the animal appropriately.

In ruminants, such as cattle, the stomach has different compartments, each having a characteristic pH. Measuring pH may assist in identifying digestive problems, such as ruminal acidosis, which is a condition wherein the pH of the rumen (the first compartment) becomes excessively acidic as a result of an unsuitable diet. Monitoring pH allows these problems to be detected and measures to be taken to prevent or treat them.

There are fixed external devices which measure the temperature of the animal's auricular region, although such devices tend to become lost as a result of the animal's natural behaviour when socialising.

There is no temperature measuring device, pH and heart rate in animals which automatically and in real time transmits the temperature data to a receiver located more than ten metres away and without human intervention. There are subcutaneous devices for measuring temperature in animals, but such devices require a human operator to be nearby in order to obtain a reading.

### EXPLANATION OF THE INVENTION

The purpose of the present invention has not been described above. On the one hand, the purpose is to provide a device for intravaginal use in females and for ruminal use in both males and females, thereby minimising the risk of loss. On the other hand, the purpose is to achieve continuous and remote monitoring of the animal's body temperature.

Once the device has been introduced into the animal, either ruminally or intravaginally, the risk of loss is extremely low, even if the animals scratch, lick themselves or perform any other action associated with their natural behaviour.

The device forming the subject matter of the invention may be used in most animals, but is particularly intended for application in cattle, pigs, goats, horses and cervids.

The remote animal-temperature monitoring device forming the subject matter of the invention comprises a housing which, in turn, comprises a hollow interior and a cover. The cover comprises a first region configured to be inserted into the housing and a second region opposite the first region. The device further comprises a core housed in the hollow interior of the housing, the core comprising a self-programmable circuit, a memory, a temperature probe responsible for measuring temperature and for sending this information to the self-programmable circuit, a heart-rate sensor configured to measure heart rate, and a pH sensor, the heart-rate sensor and the pH sensor being connected to the self-programmable circuit and being configured to send the information to said self-programmable circuit, and a battery.

In the remote animal-temperature monitoring device forming the subject matter of the invention, the first region of the housing comprises grooves wherein seals are located, the seals being configured to provide fluid-tightness.

In the remote animal-temperature monitoring device forming the subject matter of the invention, the hollow interior of the housing comprises plastic guides configured to retain the core in the hollow interior of the housing.

In the remote animal-temperature monitoring device forming the subject matter of the invention, the housing comprises an aperture at one end.

In the remote animal-temperature monitoring device forming the subject matter of the invention, the second region comprises a thread configured to allow different accessories of the device forming the subject matter of the invention to be fitted.

In the remote animal-temperature monitoring device forming the subject matter of the invention, the self-programmable circuit is configured to periodically take a reading from the temperature probe and store the values in the memory, to periodically take and/or receive a reading from the heart-rate sensor and store the values in the memory, to periodically take and/or receive a reading from the pH sensor and store the values in the memory, and to send the information from the memory, by means of a transmitter, to a receiver.

In the remote animal-temperature monitoring device forming the subject matter of the invention, the temperature probe measures a temperature range from 0°C to +125°C and has a measurement accuracy of +- 0.05°C.

In the remote animal-temperature monitoring device forming the subject matter of the invention, the transmitter uses radiofrequency at an operating frequency of 868 MHz.

### BRIEF DESCRIPTION OF THE DRAWINGS

To supplement the description provided herein and in order to assist in a better understanding of the features of the invention, a set of drawings is appended as an integral part of said description, wherein the following is shown by way of illustration and without limitation:
Figure 1 shows a view of the outer housing of the device.
Figure 2 shows a view of the cap which is inserted into the outer housing of the device.
Figure 3 shows a complete view of the device with the cap inserted.
Figure 4 shows a front view of the interior of the device with all the components forming the device.
Figure 5 shows a side view of the interior of the device with all the components forming the device.

A list of the reference numerals used in the figures accompanying this specification is provided below:
1. housing,
2. hollow interior,
3. cover,
4. core,
5. plastic guides,
6. aperture,
7. first region,
8. second region,
9. thread,
10. seals,
11. core,
12. self-programmable circuit,
13. temperature probe,
14. heart-rate sensor,
15. pH sensor,
16. battery.

### PREFERRED EMBODIMENT OF THE INVENTION

With reference to the aforementioned figures and in accordance with the numbering adopted, a preferred embodiment of the invention can be seen therein, comprising the parts and elements indicated and described below.

The device forming the subject matter of the invention comprises a housing (1) having dimensions which, in the preferred embodiment of the invention, are between 8 and 10 centimetres in length and between 2 and 2.5 cm in diameter at its widest portion. In smaller versions, the housing is 4 cm in length and 1 cm in diameter.

In the preferred embodiment of the invention, the housing (1) is spindle-shaped and is coated with a durable plastics material.

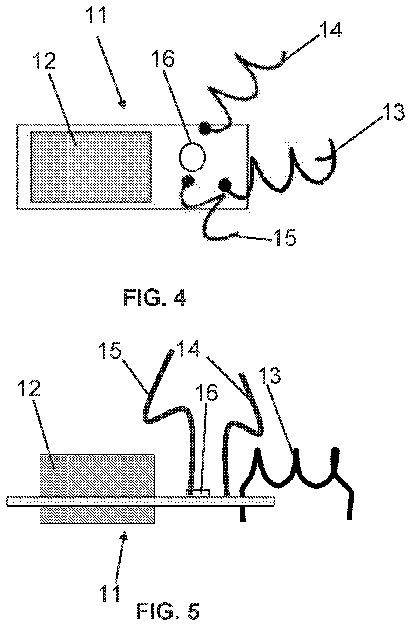

The housing (1) comprises a hollow interior (2), wherein a core (4) is housed (see Figures 4 and 5), the core (4) being retained by plastic guides (5) located in the inner region of the housing (1). At one end of the housing (1), an aperture (6) is provided which is configured to receive a cord (not shown) in order to facilitate removal of the device forming the subject matter of the invention.

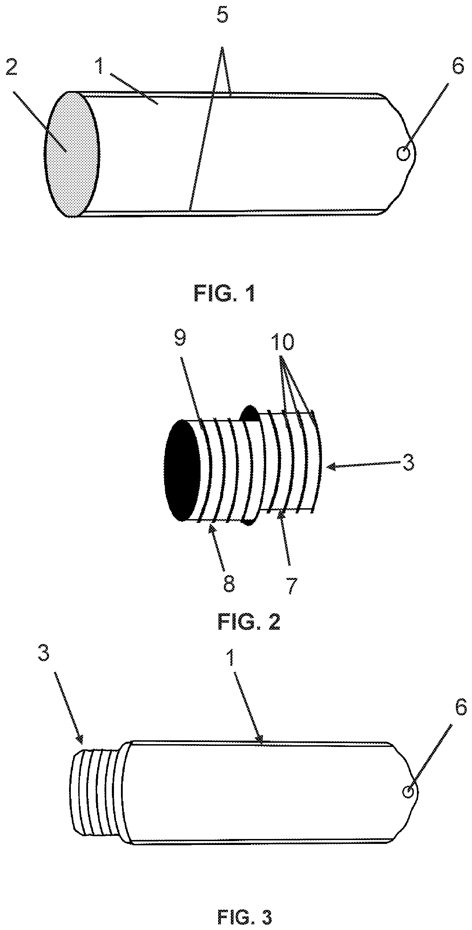

Figure 2 shows the cover (3) of the device. The cover (3) comprises a first region (7) configured to be inserted into the housing (1) and a second region (8), which comprises a thread (9) configured to allow different accessories of the device forming the subject matter of the invention to be fitted. The first region (7) comprises seals (10) located in grooves, the seals (10) being responsible for providing fluid-tightness to the interior of the device forming the subject matter of the invention. The configuration of the seals (10) provides an IP67 degree of protection against water and dust.

Figure 3 shows a view of the housing (1) with the cover (3) inserted, thereby providing a complete view of the exterior of the device forming the subject matter of the invention.

Figure 4 shows a schematic view of the core (4) of the device. The core (4) comprises a self-programmable circuit (12), which is the principal component of the device and to which the remaining components, including a memory, are connected. At the right-hand end of Figure 4, a temperature probe (13) is shown. The temperature probe (13) is responsible for measuring temperature and sends this information to the self-programmable circuit (12). A battery (16) connected to the self-programmable circuit (12) is located at the left-hand side of Figure 4. The battery (16) supplies power to the device and allows the device to be used for a number of years until replacement of the battery (16) becomes necessary.

The device forming the subject matter of the invention further comprises a heart-rate sensor (14) and a pH sensor (15), the heart-rate sensor (14) and the pH sensor (15) being connected to the self-programmable circuit (12), such that said sensors (14, 15) send the information collected by the sensors (14, 15) to the self-programmable circuit (12).

The self-programmable circuit (12) is responsible for periodically reading the temperature probe (13), the heart-rate sensor (14) and the pH sensor (15) and for storing the values in the memory.

Every hour, a transmitter (not shown) transmits the information contained in the memory, the information being received by a receiver and sent to the server.

The range of temperatures that the temperature probe (13) can measure extends from 0°C to +125°C, and the measurement accuracy thereof is +- 0.05°C.

The information stored in the self-programmable circuit (12) is sent together with a unique identifier of the device, which is also stored in the device, thereby allowing the data from each device to be associated with the animal to which the device corresponds.

Once the receiver receives the data, the receiver transmits the information via the Internet to any suitable device. The receiver (not shown) is located near the animals' drinking points, thereby ensuring constant reception of the data, since the animals drink several times a day and the device must be within range for the data to be correctly transmitted and received. Remote temperature monitoring is thereby achieved without having to handle the animal on a daily basis.

The transmission technology used is radiofrequency (RF) at an operating frequency of 868 MHz, with a transmission range of up to 150 metres under ideal conditions.

## Claims

1. Remote animal-temperature monitoring device, **characterised in that** it comprises:
- a housing (1), which in turn comprises a hollow interior (2) and a cover (), the cover comprising a first region (7) configured to be inserted into the housing (1) and a second region (8) opposite the first region (7);
- a core (4) housed in the hollow interior (2) of the housing (1),
the core (4) comprising a self-programmable circuit (12), a memory, a temperature probe (13) responsible for measuring temperature and for sending this information to the self-programmable circuit (12), a heart-rate sensor (14) configured to measure heart rate, and a pH sensor (15), the heart-rate sensor (14) and the pH sensor (15) being connected to the self-programmable circuit (12) and being configured to send the information to said self-programmable circuit (12), and a battery (16).

2. Remote animal-temperature monitoring device according to claim 1, **characterised in that** the first region (7) of the housing (1) comprises grooves wherein seals (10) are located, the seals (10) being configured to provide fluid-tightness.

3. Remote animal-temperature monitoring device according to any one of the preceding claims, **characterised in that** the hollow interior (2) of the housing (1) comprises plastic guides (5) configured to retain the core (4) in the hollow interior (2) of the housing (1).

4. Remote animal-temperature monitoring device according to any one of the preceding claims, **characterised in that** the housing (1) comprises an aperture (6) at one end.

5. Remote animal-temperature monitoring device according to any one of the preceding claims, **characterised in that** the second region (8) comprises a thread (9) configured to allow accessories of the device forming the subject matter of the invention to be fitted.

6. Remote animal-temperature monitoring device according to any one of the preceding claims, **characterised in that** the self-programmable circuit (12) is configured to:
- periodically take and/or receive a reading from the temperature probe (13) and store the values in the memory;
- periodically take and/or receive a reading from the heart-rate sensor (14) and store the values in the memory;
- periodically take and/or receive a reading from the pH sensor (15) and store the values in the memory;
- send the information from the memory, by means of a transmitter, to a receiver.

7. Remote animal-temperature monitoring device according to any one of the preceding claims, **characterised in that** the temperature probe (13) measures a temperature range from 0°C to +125°C.

8. Remote animal-temperature monitoring device according to any one of the preceding claims, **characterised in that** the transmitter uses radiofrequency at an operating frequency of 868 MHz.
